# EUROPÄISCHE PATENTSCHRIFT

(11) **EP 2 207 435 B1**
(45) Veröffentlichungstag und Bekanntmachung des Hinweises auf die Patenterteilung: **11.12.2013**
(21) Anmeldenummer: 08842146.6
(22) Anmeldetag: 15.10.2008
(51) Int. Cl.: A23L 1/30, A23L 2/52, A61K 31/05, A61K 36/87, A61K 36/45, A23L 1/302, A23L 1/304, A23L 1/305, A61K 36/82, A61K 8/365, A61K 8/49, A61K 8/60, A61Q 19/08, A61K 8/97, F27B 17/02, A23L 1/29

(54) **FUNKTIONSGETRÄNK**
FUNCTIONAL DRINK
BOISSON FONCTIONNELLE

(30) Priorität: 24.10.2007 AT 17332007
(43) Veröffentlichungstag der Anmeldung: 21.07.2010
(73) Patentinhaber: Zehethofer, Karl, 4020 Linz (AT)
(72) Erfinder: Zehethofer, Karl, 4020 Linz (AT)
(74) Vertreter: Burger, Hannes
(86) Internationale Anmeldenummer: PCT/IB2008/002733
(87) Internationale Veröffentlichungsnummer: WO 2009/053802

(56) Entgegenhaltungen:
- WO-A-00/21526
- WO-A-99/01148
- WO-A-2007/049796
- US-A1- 2006 251 608
- US-A1- 2007 122 502
- TOSCA L. ZERN, RICHARD J. WOOD, CHRISTINE GREENE, KRISTY L. WEST, YANZHU LIU, DIMPLE AGGARWAL, NEIL S. SHACHTER, MARIA FERNANDEZ: "Grape Polyphenols Exert a Cadioprotective Effect in Pre- and Postmenopausal Women by Lowering Plasma Lipids and Reducing Oxidative Stress" HUMAN NUTRITION AND METABOLISM, Bd. 135, 2005, Seiten 1911-1917, XP002519255
- BIANCA FUHRMAN AND MICAHEL AVIRAM: "Reply to Chow" THE JOURNAL OF NUTRITION, Bd. 136, 2006, Seiten 2273-2273, XP002519256
- CORDOVA A C ET AL: "The cardiovascular protective effect of red wine" 1. März 2005 (2005-03-01), JOURNAL OF THE AMERICAN COLLEGE OF SURGEONS, COLLEGE, CHICAGO, IL, US, PAGE(S) 428 - 439 , XP004764825 ISSN: 1072-7515 Seite 429, Spalte 1, Absatz 3 Seite 431, Spalte 2, Absatz 3 Seite 432; Tabellen 3,4 Seite 437, Spalte 2, Absatz 2
- STOCLET ET AL: "Vascular protection by dietary polyphenols" 1. Oktober 2004 (2004-10-01), EUROPEAN JOURNAL OF PHARMACOLOGY, ELSEVIER BV, NL, PAGE(S) 299 - 313 , XP005480425 ISSN: 0014-2999 Seite 309, Spalte 1, Absatz 2 Seite 309, Spalte 2, Absatz 2

## Beschreibung

Die Erfindung betrifft ein als wässrige Lösung flüssiges Lebensmittel, welches unter anderen Substanzen Resveratrol beinhaltet und eine sogenannte Anti-Aging-Wirkung aufweist.

Etwa seit dem Jahr 2003 finden die gesundheitlich vorteilhaften Wirkungen von Resveratrol, seinen trans-Isomeren (des Weiteren vereinfachend gemeinsam mit "Resveratrol," bezeichnet) sowie die gemeinsam mit Resveratrol im Extrakt von roten Trauben vorkommenden Stoffe hohe Beachtung.

Resveratrol ("5-[(E)-2-(4-hydroxyphenyl)-ethenyl]benzene-1,3-diol", chemische Summenformel: C₁₄H₁₂O₃) ist ein Polyphenol welches zur Klasse der Stilbene zählt und zur Gruppe der Phytoalexine gehört. Es ist ein besonders hoch wirksamer Radikalfänger, und damit in der Lage oxidativen Stress zu minimieren. Es schützt vor Gefäßverkalkung, verlangsamt das Wachstum von Krebszellen bzw. tötet diese ab, senkt das Herzinfarktrisiko, wirkt dem Alterungsprozess entgegen und verlängert die Lebenszeit, ist entzündungshemmend, senkt den Cholesterinspiegel, beugt Prostata-Krebs vor, senkt das Risiko eines Gehirnschlages, senkt das Risiko an Dickdarmkrebs zu erkranken, vermindert das Risiko an Alzheimer zu erkranken und weist antimikrobielle und anti-virale Eigenschaften auf.

Die EP1648437 A2 (WO 20051002672 A2) schlägt vor, durch Einwirkung von Resveratrol in Kombination mit Stoffen wie beispielsweise Niacin auf eukariotische Zellen die Expression der Sirtuin-Gene wie SIRTl zu fördern, und damit eine lebensverlängernde, Anti-Aging-Wirkung zu erzielen. Das kann sowohl in vitro erfolgen, also auch auf Zellen in Lebewesen wie Tieren oder Menschen. Die Anwendung für Menschen wird dabei nur im medizinischen Bereich vorgeschlagen, beispielsweise als Hilfsstoff bei der Chemotherapie zur Krebsbehandlung.

Die US 6, 368, 617 B1 schlägt einen Diätzusatzstoff zur Vermeidung von oxidativen Stress für den Organismus von erwachsenen Menschen vor. Der Diätzusatzstoff enthält neben Resveratrol u.a. auch Fruchtzucker und Zitronensäure und bevorzugt eine Mischung aus zerriebenen Kräutern. Bevorzugt wird er als Pulver, typischerweise als feines grünes Pulver angeboten, welches zur Konsumation in Wasser eingerührt und mit diesem getrunken wird.

Die DE 102 23 262 A1 beschreibt einen Kaffee-Ersatzstoff. Es soll die belebende Wirkung von Kaffee aufweisen, nicht jedoch schädliche Nebenwirkungen wie beispielsweise das Herstellen von suchtartiger Abhängigkeit. Das wasserlösliche Mittel enthält neben Ginkobestandteilen in einzelnen vorgeschlagenen Beispielen Grüntee-Extrakt, Traubenkernextrakt und Fruchtzucker.

In der WO 02/081651 A2 wird ein Getränk vorgeschlagen, welches die vorteilhaften Eigenschaften von Rotwein im wesentlichen auf Grund der darin enthaltenen Polyphenole auf das Herz-Kreislaufsystem aufweisen soll, ohne deswegen Alkohol zu enthalten. Neben Resveratrol enthält das Getränk auch Katechin aus dem Extrakt von grünem Tee.

Die WO 2004/105517 A1 beschreibt ein entzündungshemmendes Getränk, welches neben Resveratrol auch Zitronensäure, Vitamin E, Vitamin C und Beta-Carotin enthält.

Die WO 2007/049796 A2 schlägt ein Anti-Aging-Mittel vor, in welchem Resveratrol und Extrakt von *Blättern von Weinreben, bevorzugt von Weinreben, an denen rote Trauben wachsen,* angewendet werden. Es werden Rezepte für ein Gelee und für Getränke vorgeschlagen, in denen daneben auch Zitronensäure und Fruchtzucker enthalten sind. Die Anti-Aging-Wirkung baut sehr isoliert auf Resveratrol auf und ist damit relativ eng. Für die Anwendung als unkompliziert haltbares, handhabbares und genießbares Getränk sind zusätzliche Additive erforderlich.

Die US 2007/0122502 A1 schlägt einen Anti-Aging-Saft für Frauen vor allem ab einem Alter von 35 Jahren vor, welcher neben dem Saft der Noni-Frucht und Bestandteilen des Hahnenfußgewächses "Trauben-Silberkerze" (Cimicifuga racemosa; als Arznei hat es östrogenähnliche Eigenschaften), neben einer Chrom- und einer Kalziumverbindung auch eine pflanzliche Quelle von Trans-Resveratrol enthält. Um trotz der geschmacklich problematischen Zugaben einen genießbaren Saft zu erhalten, werden weitere Zusätze wie Fruchtsäfte und Zitronensäure zugeschlagen. *Als Quelle von Trans-Resveratrol werden ein bis drei Gewichtsprozent blaue Trauben zugeschlagen. Die ebenfalls zugeschlagene Substanz "exberry grape" betrifft einen aus Pflanzen gewonnen Farbstoff und kein Traubenfruchtkonzentrat.*

In der WO 2007/112366 A2 wird vorgeschlagen, Resveratrol, welches aus verschiedenen Pflanzen gewonnen werden kann, auf Grund seiner vielfältig gesundheitsfördernden Wirkung an Tiere zu verfüttern. Für Menschen anwendbare Darreichungsformen werden nicht behandelt.

*In der Abhandlung*
TOSCA L. ZERN, RICHARD J. WOOD, CHRISTINE GREENE, KRISTY L. WEST, YANZHU LIU, DIMPLE AGGARWAL, NEIL S. SHACHTER, MARIA FERNANDEZ: "Grape Polyphenols Exert a Cadioprotective Effect in Pre- and Postmenopausal Women by Lowering Plasma Lipids and Reducing Oxidative Stress" HUMAN NUTRITION AND METABOLISM, Bd. 135, 2005, Seiten 191 1-1 91 7, XP002519255
*- wird die Wirkung eines lyophilisierten Traubenextraktes-der zu 92% aus Zucker besteht - für Frauen vor und während der Menopause beschrieben. Es wird nicht angedacht, Resve-ratrol (als isolierte Substanz), oder andere Zusatzstoffe extra beizumengen. Die Rezeptur wird als getrocknetes Pulver bereitgestellt, Zum Trinken wird sie in Wasser angerührt.*

*In der Abhandlung*
BIANCA FUHRMAN AND MICAHEL AVIRAM: "Reply to Chow" THE JOURNAL OF NUTRITION, Bd. 136, 2006, Seiten 2273-2273*, XP002519256* - *wird die Wirkung von gefriergetrocknetem Traubenpulver beschrieben*

*In der Abhandlung*
CORDOVA A C ET AL: "The cardiovascular protective effect of red wine" 1. März 2005 (2005-03-01), JOURNAL OF THE AMERICAN COLLEGE OF SURGEONS, COLLEGE, CHICAGO, IL, US, PAGE (S) 428-439 , XP004764825 ISSN: 1 072-751 5 *werden vorteilhafte Wirkungen von Rotwein beschrieben.*

*Die Abhandlung*
STOCLET ET AL: "Vascular protection by dietary polyphenols" 1. Oktober 2004 (2004-1 0-OI), EUROPEAN JOURNAL OF PHARMACOLOGY, ELSEVIER BV, NL, PAGE(S) 299 -31 3 ,XP005480425 ISSN: 001 4-2999
- *gibt an Aussage bezüglich der vorliegenden Anmeldung etwas verallgemeinert die Lehren aus den beiden zuvor beschriebenen Abhandlungen wieder.*

*Die* WO 99/01148 A *gibt zu der vorliegenden Anmeldung auch etwa die gleiche Lehre wieder. Als Quelle von Resveratrol, wird getrockneter Extrakt von Wein verwendet, welcher auch wieder verflüssigt werden kann. Ein wichtiger Zusatz dabei ist Bierhefe.*

*Die* WO 00/21526 A *befasst sich mit den vorteilhaften Wirkungen von Traubenextrakt.*

*Die* US 2006/0251608 A1 *beschreibt eine Präparatkombination, welche aus zwei unterschiedlichen, in einem zeitlichen Abstand von 12 Stunden einzunehmenden Tabletten sowie aus einer topisch aufzutragenden Creme besteht. Die erste Tablette enthält die Stoffe L-Carnitin, Rotweintraubenpolyphenol-Extrakt, Grüntee Extrakt, Vitamin C, Vitamin E, eine geringe Menge Konzentrat aus roten Beerenfrüchten sowie Beta-Carotin. Die zweite Tablette enthält Resveratrol sowie Ubichinon (Coenzym Q10) und Selen. Die Präparatkombination dient dazu gealterte, faltige Haut zu kurieren.*

In Kenntnis dieses Standes der Technik hat sich der Erfinder die Aufgabe gestellt, eine Rezeptur für ein als wässrige Lösung flüssiges, drinkbares Lebensmittel auf Basis Resveratrol bereitzustellen, welches die Entfaltung der gesundheitsfördernden Auswirkungen, insbesondere die Anti-Aging-Wirkung von Resveratrol bestmöglich zulassen soll und darüber hinaus weitere, in Richtung "Anti-Aging" wirkende Stoffe enthält.

Weiters ist wichtig, dass das Lebensmittel gut handhabbar ist. Das heißt:
- die enthaltenen Wirkstoffe müssen als Mischung ausreichend gut in Wasser löslich, sein, sodass es zu keinen Ausflockungen und/oder Anhaftungen oder Ablagerungen am Gebinde kommt,
- das Lebensmittel muss als Fertiggetränk ohne weitere Konservierungsmittel und Stabilisatoren auch ungekühlt gut haltbar und transportierbar sein,

Die wesentlichen, für die Verwendung als Lebensmittel im Allgemeinen vorteilhaften Eigenschaften wie Anmutung (Farbe, Viskosität,..), Geruch, Geschmack, Haltbarkeit, Stabilität gegen Entmischung etc. sollen in höchstmöglichem Ausmaß nicht durch speziell daraufhin ausgerichtete Lebensmittelzusätze erreicht werden, sondern durch möglichst naturnahe, ohnedies für die Anti-Aging-Wirkung erforderlichen Inhaltsstoffe.

Die erfindungsgemäße Lösung besteht in der Bereitstellung eines Verfahrens gemäß Anspruch 1.

Ein Saftkonzentrat aus roten, genießbaren Beerenfrüchten wird zumindest in jener Menge zugegeben, dass es den Geschmack der Rezeptur prägt. Vorzugsweise soll das Saftkonzentrat aus roten genießbaren Beerenfrüchten auch die Farbe der Rezeptur prägen.

Als rote Beerenfrüchte kommen beispielsweise Erd-, Him-, Brom-, Preisel-, Heidel-, rote Johannisbeeren, Kirschen, Weichsel, Holunder, rote Weintrauben in Frage.

Die roten Beerenfrüchte sind einerseits aus Darstellungsgründen vorteilhaft, da sie die Assoziationsverbindung zu Rotweintrauben erleichtern, welche die wesentliche Quelle für Resveratrol und ähnliche vorteilhafte Wirksubstanzen bilden. Weiters sind diese Beerenfrüchte vom Geschmack her überwiegend sehr beliebt.

Von ganz wesentlicher Bedeutung an den roten Beerenfrüchten ist aber auch, dass diese Beerenarten tatsächlich Inhaltsstoffe aufweisen, welche (bekanntermaßen) eine positive Wirkung auf das Gefäßsystem innehaben.

Rote Beerenfrüchte wie Holunder- und Heidelbeeren sind bereits seit Jahrhunderten in Europa und in Nordamerika in der Naturheilkunde in Verwendung. Aktuelle Studien der Indiana University School of Medicine haben nun die Wirkung ihrer Inhaltsstoffe, Flavonoide, Polyphenole und Anthocyane belegt. Besonders den Anthocyanen, die für die rote und blaue Färbung der Früchte zuständig sind, wird eine positive Wirkung auf die menschliche Gesundheit zugesprochen.

Als besonders vorteilhaft hat sich eine Mischung aus Saftkonzentraten von Holunderbeeren und schwarzen Johannisbeeren herausgestellt. Diese enthalten neben der vorteilhaften Farbe auch Polyphenole, Anthocyane und Flavonoide. Anthocyane, wie der Farbstoff des Holunders, verbessern die Sehvorgänge, sind entzündungshemmend und wirken gefäßschützend. Zusätzlich habe sie eine hohe antioxidative Wirkung und vermindern signifikant das Schlaganfallrisiko.

Holunder verlangsamt den Alterungsprozess der Zellen durch Schutz der Zellmembranen durch freie Radikale und wirkt als Antioxidans. Holunder ist reich an Vitamin C, B, Fruchtsäuren, ätherischen Ölen (wirken leicht schweißtreibend und schleimlösend), Flavonoiden und dem farbgebenden Anthocyan. Holunder hilft gegen Erkältungen, Nieren- und Blasenleiden und Magenbeschwerden. Weiters enthält der schwarze Holunder einen violetten Farbstoff, das Sambucyanin, dass ein sekundärer Pflanzenstoff ist und als Antioxidans das Risiko von Krebs und Herz-Kreislauferkrankungen senkt.

Johannisbeeren enthalten neben Wasser, Proteinen, Kohlenhydraten, Fettsäuren auch Mineralien und Anthocyane, Flavonoide und Catechin (wie im Grüntee). Daneben kommen Vitamin C, Zitronensäure und β-Caryophyllen vor, β-Caryophyllen ist ein CB₂Cannabinoid und wirkt enzündungs-, schmerz-, atherosklerosis- und osteoporosishemmend.

Die Verwendung von roten Beerenfrüchten ist also nicht nur aus optisch-assoziativen Gründen und aus geschmacklichen Gründen, sondern auch aus wissenschaftlich argumentierbaren, gesundheitlichen Gründen vorteilhaft.

Neben den genannten Stoffen sind als weitere Inhaltsstoffe in dem Lebensmittel vorteilhaft:
Beta-Carotin; Es ist eine Vorstufe von Vitamin A und wird im Körper nur bei Bedarf in Vitamin A umgewandelt, das bedeutet, dass es auch bei höherer Dosierung nicht schädlich ist. Vitamin A ist wichtig für den Sehvorgang, für den Aufbau von dermalen Strukturen, fördert die Bildung von Blutkörperchen und spielt eine wichtige Rolle im Stoffwechsel bei der Proteinsynthese. Darüber hinaus ist Carotin ein zugelassener, rötlich färbender Lebensmittelfarbstoff.
L-Carnitin: - ist eine natürlich vorkommende, vitaminähnliche Substanz. Es spielt eine wichtige Rolle im Energiestoffwechsel menschlicher, tierischer und pflanzlicher Zellen. In Verbindung mit Sport kann L-Carnitin als Fettverbrennungsmittel wirken.
Ubichinon Coenzym Q₁₀: - ist strukturell verwandt mit Vitamin K und Vitamin E. Es wird in den Mitochondrien als Elektronen- und Protonencarrier in der zellulären Atmungskette gebraucht und wirkt als Antioxidans. Ein Ubichinon-Mangel entsteht durch altersbedingte Abnahme der Ubichinon-Konzentration, körperlicher Anstrengung, Stress, erhöhtem Alkohol- und Nikotinkonsum sowie Krankheiten. Manche cholesterinsenkende Medikamente (die sogenannten "Statine") hemmen die körpereigene Produktion von Ubichinon. Durch Ubichinon als Nahrungsergänzung aufgenommen kann dieser Mangel vermieden werden.
Selen: - kann als Radikalfänger eine wichtige Rolle beim Schutz der Zellmembranen vor oxidativer Zerstörung spielen. Niacin (Vitamin B3) entfaltet seine Wirkung viel besser im Zusammenspiel mit Vitamin C und E und steigert die genexpressionsfördernde Wirkung auf die Sirtuin-Gene von Resveratrol. Auch mit weiteren Effekten bewirkt es eine erhebliche Verbesserung der "Anti-Aging-Wirkung:

Es wirkt cholesterin- und triglycerinsenkend, reduziert Fettablagerungen im Gewebe, verbessert die Funktion der roten Blutkörperchen und deren Sauerstofftransportkapazität, was eine verbesserte Gehirnfunktion bewirkt. Niacin reduziert das cardiovaskulare Risiko, wirkt als atheroprotektives Agens. Niacin ist an 200 Reaktionen im Stoffwechsel beteiligt. Generell Vermehrung, Schutz und Reparatur der Zellen, der durch Oxidation entstandenen Schäden, senkt das gesamt Cholesterin, entfaltet antioxidative Wirkung besonders in der Leber, spielt eine wichtige Rolle in der Energieproduktion, Blutzucker-Regulierung und Entgiftung von Umweltschadstoffen über das Cytochrom P 450-System. Niacin greift in eine Vielzahl von Auf und Abbaufunktionen im Amino -Kohlehydrat- Fett, Steroid-, Vitamin- und Energiestoffwechsel ein.

Als Beispiel für eine vorteilhafte Rezeptur sei genannt:

| | | |
|---|---|---|
| Fruchtzucker | 110 | g |
| Johannisbeere Saftkonzentrat. | 20 | g |
| Holunderbeere Saftkonzentrat | 14 | g |
| Zitronensäure | 7 | g |
| L-Carnitin | 1 | g |
| Rotweintraubenpolyphenol-Extrakt | 200 | mg |
| Grüntee Extrakt | 200 | mg |
| Vitamin C | 120 | mg |
| Resveratrol | 100 | mg |
| Niacin | 36 | mg |
| Vitamin E | 36 | mg |
| Ubichinon Coenzym Q₁₀ | 10 | mg |
| beta-Carotin | 4 | mg |
| Selen | 1,5 | µg |

Die Rezeptur ist problemlos in der auf eine Gesamtmasse von 1 kg noch fehlenden Masse Wasser lösbar. Sie ist bei Normaltemperatur problemlos lager- und transportierbar. Angesichts der vielen, zueinander teilweise recht unterschiedlicher Inhaltsstoffe ist es überraschend, dass es zu keinerlei störenden Entmischungen oder Ausfällungen oder sonstigen unvorteilhaften Wechselwirkungen kommt.

In der in Wasser gelösten Form stellt die Rezeptur ein wohlschmeckendes, appetitlich aussehendes, sehr gesundes Fertiggetränk dar, welches problemlos von Menschen jeden Geschlechts und jeder Altersgruppe täglich getrunken werden kann. Das Getränk ist einfach durch Zusammenwägen und Rühren der entsprechenden Komponenten herstellbar und von seinen Eigenschaften über herkömmliche Getränke-Vertriebskanäle ungekühlt verteil- und verkaufbar. Wenn neben dem Fruchtzucker kein zusätzlicher Kristallzucker (Saccharose) beigemengt ist, so ist dieses Getränk auch für Diabetiker problemlos geeignet.

Neben dem Erfüllen der, der Erfindung zu Grunde liegenden Aufgabenstellung weist das Lebensmittel an weiteren Vorteilen auf:
- Es bedarf keiner separaten Stoffe die für ein vorteilhaftes Mundgefühl erforderlich sind. "Mundgefühl" betrifft beispielsweise folgende technischen Eigenschaften eines Lebensmittels: Dichte, Haftung, Viskosität, Feuchtigkeitseigenschaften, Körnigkeit.
- Das Lebensmittel ist für Diabetiker geeignet, da neben Fruchtzucker keine künstlichen oder natürlichen Süssungsmittel erforderlich sind.
- Das Lebensmittel ist für Personen, die an Laktoseintoleranz leiden, geeignet.
- Das Lebensmittel kommt ohne Gelatin aus.
- Das Lebensmittel ist einfach in PET-Flaschen haltbar und transportierbar.
- Das Lebensmittel ist ein ausgezeichneter Durstlöscher, da es zu rund 85% aus reinem Wasser besteht und einen fruchtigen, erfrischenden Geschmack aufweist.
- Die verschiedenen Wirkstoffe sind zueinander kreuzverträglich, das heißt sie vermindern sich nicht in ihrer jeweiligen gewünschten Wirkung, sondern sie verstärken bzw. ergänzen sich gegenseitig. Das betrifft nicht nur die Eigenschaften als gut genießbares und gut handhabbares Getränk, sondern auch die erwünschte Breite von Einzelwirkungen zwecks Bildung eines Lebensmittels, dass die Bezeichnung "Anti-Aging-Lebensmittel" wirklich verdient.

Es sei darauf hingewiesen, dass das beschriebene Lebensmittel nicht nur für Menschen sondern auch für andere Säugetiere wie beispielsweise Pferde, Hunde und Kamele im Wesentlichen die gleichen vorteilhaften Wirkungen aufweist. In diesem Sinn sind in den nachfolgenden Patentansprüchen unter dem Begriff "Lebensmittel" nicht nur Mittel zu verstehen, welche Menschen oral aufnehmen, sondern auch Futtermittel von Säugetieren.

## Patentansprüche

1. Verfahren zur Herstellung eines Getränks durch Zusammenwägen und Rühren der Stoffe Fruchtzucker, Resveratrol, Rotweintraubenpolyphenol-Extrakt und Konzentrat aus roten Beerenfrüchten sowie gegebenenfalls Zitronensäure, L-Carnitin, Grüntee Extrakt, Vitamin C, Niacin, Vitamin E, Beta-Carotin, Ubichinon Coenzym Q10 und Selen, **dadurch gekennzeichnet, dass** je kg Trockensubstanz diese Stoffe in folgenden Mengengrenzen zugesetzt werden:
| **Stoff** | **Minimum** | | **Maximum** |
|---|---|---|---|
| Fruchtzucker | 200g | - | 800g |
| Resveratrol | 0,4g | - | 2g |
| Rotweintraubenpolyphenol-Extrakt | 0,5g | - | 3g |
| Konzentrat aus roten Beerenfrüchten | 100g | - | 300g |
| Zitronensäure | 0g | - | 100g |
| L-Carnitin | 0g | - | 12g |
| Grüntee Extrakt | 0g | - | 3g |
| Vitamin C | 0g | - | 2g |
| Niacin | 0 g | - | 0,5g |
| Vitamin E | 0 g | - | 0,5g |
| Beta-Carotin | 0 mg | - | 50mg |
| Ubichinon Coenzym Q10 | 0 mg | - | 100mg |
| Selen | 0 µg | - | 30 µg |
und dass die gesamte Trockensubstanz in eine einzige, gemeinsame wässrige Lösung gebracht wird.

2. Verfahren nach Anspruch 1, **dadurch gekennzeichnet, dass** die roten Beerenfrüchte ausgewählt werden aus einer Gruppe bestehend aus Erdbeeren, Himbeeren, Brombeeren, Preiselbeeren, Heidelbeeren, rote Johannisbeeren, Kirschen, Weichseln, Holunder.

3. Verfahren nach Anspruch 1, **dadurch gekennzeichnet, dass** als Konzentrat aus roten Beerenfrüchten eine Mischung aus Saftkonzentraten von Holunder und schwarzen Johannisbeeren verwendet wird.

4. Verfahren nach Anspruch 1 oder Anspruch 2, **dadurch gekennzeichnet, dass** je kg Trockensubstanz folgende Stoffe in folgenden Mengengrenzen zugesetzt werden:
| **Stoff** | **Minimum** | | **Maximum** |
|---|---|---|---|
| Fruchtzucker | 680g | - | 760g |
| Johannisbeere Saftkonzentrat. | 100g | - | 160g |
| Holunderbeere Saftkonzentrat | 70g | - | 115g |
| Zitronensäure | 35g | - | 55g |
| L-Carnitin | 5g | - | 8g |
| Rotweintraubenpolyphenol-Extrakt | 1,1g | - | 1,5g |
| Grüntee Extrakt | 1,1g | - | 1,5g |
| Vitamin C | 0,6g | - | 0,9g |
| Resveratrol | 0,6g | - | 0,7g |
| Niacin | 0, 2 g | - | 0,3g |
| Vitamin E | 0,2g | - | 0,3g |
| Ubichinon Coenzym Q 10 | 45mg | - | 85mg |
| Beta-Carotin | 20mg | - | 30mg |
| Selen | 0 µg | - | 15 µg |

5. Verfahren nach Anspruch 1, **dadurch gekennzeichnet, dass** keine Saccharose enthalten ist.

6. Verfahren nach Anspruch 1, **dadurch gekennzeichnet, dass** die Stoffe in folgenden Mengen zugesetzt werden.
| | | |
|---|---|---|
| Fruchtzucker | 110 | g |
| Johannisbeere Saftkonzentrat. | 20 | g |
| Holunderbeere Saftkonzentrat | 14 | g |
| Zitronensäure | 7 | g |
| L-Carnitin | 1 | g |
| Rotweintraubenpolyphenol-Extrakt | 200 | mg |
| Grüntee Extrakt | 200 | mg |
| Vitamin C | 120 | mg |
| Resveratrol | 100 | mg |
| Niacin | 36 | mg |
| Vitamin E | 36 | mg |
| Ubichinon Coenzym Q₁₀ | 10 | mg |
| beta-Carotin | 4 | mg |
| Selen | 1,5 | µg |
und den Rest auf eine Gesamtmasse von 1 kg Wasser bildet, in dem diese Stoffe gelöst sind.

## Claims

1. Method for producing a drink by mixing and stirring the ingredients fructose, resveratrol, red wine grapes polyphenol extract and concentrate of red berry fruits as well as, optionally citric acid, L-carnitine, green tea extract, vitamin C, niacin, vitamin E, beta-carotene, ubiquinone coenzyme Q10 and selenium, **characterized in that** these substances in the following quantity limits are added to one kilogram dry matter:
| **Substance** | **Minimum** | | **Maximum** |
|---|---|---|---|
| Fructose | 200g | - | 800g |
| Resveratrol | 0.4g | - | 2g |
| Red wine grapes polyphenol extract | 0.5g | - | 3g |
| Concentrate of red berry fruits | 100g | - | 300g |
| Citric acid | 0g | - | 100g |
| L-carnitine | 0g | - | 12g |
| Green tea extract | 0g | - | 3g |
| Vitamin C | 0g | - | 2g |
| Niacin | 0g | - | 0.5g |
| Vitamin E | 0g | - | 0.5g |
| Beta-carotene | 0mg | - | 50mg |
| Ubiquinone coenzyme Q10 | 0mg | - | 100mg |
| Selenium | 0µg | - | 30µg |
and that the whole of the dry substance is put into one single common aqueous solution.

2. Method according to claim 1, **characterized in that** the red berry fruits are selected from a group consisting of strawberries, raspberries, blackberries, cranberries, blueberries, red currant, cherries, sour cherries, elder.

3. Method according to claim 1, **characterized in that** a mixture of concentrated juice of elder and black currant is used as a concentrate of red berry fruits.

4. Method according to claim 1 or claim 2, **characterized in that** the following substances in the following quantity limits are added per kilogram dry matter:
| **Substance** | **Minimum** | | **Maximum** |
|---|---|---|---|
| Fructose | 680g | - | 760g |
| Black currant concentrated juice | 100g | - | 160g |
| Elderberry concentrated juice | 70g | - | 115g |
| Citric acid | 35g | - | 55g |
| L-carnitine | 5g | - | 8g |
| Red wine grapes polyphenol extract | 1.1g | - | 1.5g |
| Green tea extract | 1.1g | - | 1.5g |
| Vitamin C | 0.6g | - | 0.9g |
| Resveratrol | 0.6g | - | 0.7g |
| Niacin | 0.2g | - | 0.3g |
| Vitamin E | 0.2g | - | 0.3g |
| Ubiquinone coenzyme Q10 | 45mg | - | 85mg |
| Beta-carotene | 20mg | - | 30mg |
| Selenium | 0µg | - | 15µg |

5. Method according to claim 1, **characterized in that** no sucrose is contained.

6. Method according to claim 1, **characterized in that** the substances are added in the following quantities
| | | |
|---|---|---|
| Fructose | 110 | g |
| Black currant concentrated juice | 20 | g |
| Elderberry concentrated juice | 14 | g |
| Citric acid | 7 | g |
| L-carnitine | 1 | g |
| Red wine grapes polyphenol | | |
| extract | 200 | mg |
| Green tea extract | 200 | mg |
| Vitamin C | 120 | mg |
| Resveratrol | 100 | mg |
| Niacin | 36 | mg |
| Vitamin E | 36 | mg |
| Ubiquinone coenzyme Q10 | 10 | mg |
| Beta-carotene | 4 | mg |
| Selenium | 1.5 | µg |
with the rest to a total amount of 1 kilogram is water, wherein these substances are dissolved.

## Revendications

1. Procédé de préparation d'une boisson par pesée ensemble et agitation des produits fructose, resvératrol, extrait de polyphénols de raisins rouges et concentré de baies et drupes rouges, ainsi que, le cas échéant, acide citrique, L-camitine, extrait de thé vert, vitamine C, niacine, vitamine E, bêta-carotène, ubiquinone (coenzyme Q10) et sélénium, **caractérisé en ce que** par kg de substance sèche, ces produits sont ajoutés dans les intervalles de quantité suivants :
| **Substance** | **Minimum** | | **Maximum** |
|---|---|---|---|
| Fructose | 200 g | - | 800 g |
| Resvératrol | 0,4 g | - | 2 g |
| Extrait de polyphénols de raisins rouges | 0,5 g | - | 3 g |
| Concentré de baies rouges | 100 g | - | 300 g |
| Acide citrique | 0 g | - | 100 g |
| L-carnitine | 0 g | - | 12 g |
| Extrait de thé vert | 0 g | - | 3 g |
| Vitamine C | 0 g | - | 2 g |
| Niacine | 0 g | - | 0,5 g |
| Vitamine E | 0 g | - | 0,5 g |
| Bêta-carotène | 0 mg | - | 50 mg |
| Ubiquinone | 0 mg | - | 100 mg |
| Sélénium | 0 µg | - | 30 µg |
et **en ce que** l'ensemble de la substance sèche est amené dans une unique solution aqueuse commune.

2. Procédé selon la revendication 1, **caractérisé en ce que** les baies ou drupes rouges sont choisies parmi un groupe consistant en les fraises, les framboises, les mûres, les airelles rouges, les myrtilles, les groseilles, les cerises, les merises, les baies de sureau.

3. Procédé selon la revendication 1, **caractérisé en ce que** comme concentré de baies ou drupes rouges est utilisé un mélange de concentrés de jus de baie de sureau et de cassis.

4. Procédé selon la revendication 1 ou la revendication 2, **caractérisé en ce que** par kg de substance sèche les produits suivants sont ajoutés dans les intervalles de quantité suivants :
| **Substance** | **Minimum** | | **Maximum** |
|---|---|---|---|
| Fructose | 680 g | - | 760 g |
| Concentré de jus de groseille | 100 g | - | 160 g |
| Concentré de jus de sureau | 70 g | - | 115 g |
| Acide citrique | 35 g | - | 55 g |
| L-carnitine | 5 g | - | 8 g |
| Extrait de polyphénols de raisins rouges | 1,1 g | - | 1,5 g |
| Extrait de thé vert | 1,1 g | - | 1,5 g |
| Vitamine C | 0,6 g | - | 0,9 g |
| Resvératrol | 0,6 g | - | 0,7 g |
| Niacine | 0,2 g | - | 0,3 g |
| Vitamine E | 0,2 g | - | 0,3 g |
| Ubiquinone (coenzyme Q 10) | 45 mg | - | 85 mg |
| Bêta-carotène | 20 mg | - | 30 mg |
| Sélénium | 0 µg | - | 15 µg |

5. Procédé selon la revendication 1, **caractérisé en ce qu'**il n'est pas contenu de saccharose.

6. Procédé selon la revendication 1, **caractérisé en ce que** les produits sont ajoutés en les quantités suivantes :
| | | |
|---|---|---|
| Fructose | 110 | g |
| Concentré de jus de groseille | 20 | g |
| Concentré de jus de sureau | 14 | g |
| Acide citrique | 7 | g |
| L-carnitine | 1 | g |
| Extrait de polyphénols de raisins rouges | 200 | mg |
| Extrait de thé vert | 200 | mg |
| Vitamine C | 120 | mg |
| Resvératrol | 100 | mg |
| Niacine | 36 | mg |
| Vitamine E | 36 | mg |
| Ubiquinone (coenzyme Q 10) | 10 | mg |
| Bêta-carotène | 4 | mg |
| Sélénium | 1,5 | µg |
et **en ce que** le reste jusqu'à une masse totale de 1 kg est constitué d'eau, dans laquelle ces produits sont dissous.
